# EUROPEAN PATENT SPECIFICATION

(11) **EP 1 450 806 B1**
(45) Date of publication and mention of the grant of the patent: **29.04.2009**
(21) Application number: 02783922.4
(22) Date of filing: 06.11.2002
(51) Int. Cl.: A61K 31/496, A61P 3/04

(54) **USE OF SULFONAMIDE DERIVATIVES IN THE TREATMENT OF OBESITY OR FOR THE REDUCTION OF FOOD INTAKE**
VERWENDUNG VON SULFONAMID-DERIVATEN BEI DER BEHANDLUNG VON ADIPOSITAS ODER ZUR VERRINGERUNG DER NAHRUNGSAUFNAHME
UTILISATION DE DERIVES SULFONAMIDE DANS LE TRAITEMENT DE L'OBESITE OU POUR LA REDUCTION DE PRISE D'ALIMENTS

(30) Priority: 09.11.2001 SE 0103767; 13.02.2002 US 356890 P
(43) Date of publication of application: 01.09.2004
(73) Proprietor: Biovitrum AB (publ), 112 76 Stockholm (SE)
(72) Inventor: CALDIROLA, Patrizia, S-756 46 Uppsala (SE)
(86) International application number: PCT/SE2002/002019
(87) International publication number: WO 2003/039547

(56) References cited:
- WO-A1-94/21619
- WO-A1-98/27081
- WO-A1-99/44609
- WO-A2-01/32646
- WO-A2-99/42465
- BENTLEY J.C. ET AL.: 'Effect of the 5-HT6 antagonist, Ro 04-6790 on food consumption in rats trained to a fixed feeding regime' BRITISH JOURNAL OF PHARMACOLOGY vol. 126, no. SUPPL., 1999, page 66P, XP002960509
- DOURISH COLIN T.: 'Multiple serotonin receptors: opportunities for new treatments for obesity' OBESITY RESEARCH vol. 3, no. SUPPL. 4, November 1995, pages 449S - 462S, XP002948186

## Description

### TECHNICAL FIELD

The present invention relates to the use of sulfonamides and their derivatives, which bind selectively to 5-HT₆ receptors, in the manufacture of a medicament for the treatment of obesity or for the reduction of food intake.

### BACKGROUND ART

Obesity is a condition characterized in an increase in body fat content resulting in excess body weight above accepted norms. Obesity is the most important nutritional disorder in the western world and represents a major health problem in all industrialized countries. This disorder leads to increased mortality due to increased incidences of diseases such as cardiovascular disease, digestive disease, respiratory disease, cancer and NIDDM (type II diabetes). Searching for compounds that reduce body weight has been going on for many decades. One line of research has been activation of serotonergic systems, either by direct activation of serotonin receptor subtypes or by inhibiting serotonin reuptake. The exact receptor subtype profile required is however not known.

Serotonin (5-hydroxytryptamine or 5-HT), a key transmitter of the peripheral and central nervous system, modulate a wide range of physiological and pathological functions, including anxiety, sleep regulation, aggression, feeding and depression. Multiple serotonin receptor subtypes have been identified and cloned. One of these, the 5-HT₆ receptor, was cloned by several groups in 1993 (Ruat et al. (1993) Biochem. Biophys. Res. Commun., 193: 268-276; Sebben et al. (1994) NeuroReport 5: 2553-2557) This receptor is positively coupled to adenylyl cyclase and displays affinity for antidepressants such as clozapine. Recently, the effect of 5-HT₆ antagonist and 5-HT₆ antisense oligonucleotides to reduce food intake in rats has been reported (Bentley et al. (1999) Br. J. Pharmac. Suppl 126: P66; Bentley et al. (1997) J. Psychopharmacol. Suppl. A64: 255).

WO01/32646 disclose sulfonamide compounds as ligands selective for the 5-HT₆ receptors, and of proposed value in the treatment or prevention of CNS disorders, including Alzheimer's disease, Parkinson's disease, schizophrenia, depression and anxiety. However, it has not been disclosed that such derivatives are useful for the treatment of obesity.

WO94/21619 relates to naphthalene derivatives that are selective for the 5-HT₁ receptors and suggested useful in the treatment of obesity.

### BRIEF DESCRIPTION OF THE DRAWINGS

Figure 1 is a graph depicting the effect on food intake in obese mice by administration of 5-chloro-3-methyl-benzo[b]thiophene-2-sulphonic acid (4-[4-methylpiperazin-1-yl] quinolin-6-yl)amide.
Figure 2 is a graph depicting the effect on food intake in obese mice by administration of 4-*n*-butyl-N-(4-piperazin-1-yl-quinolin-6-yl)benzenesulphonamide.

### DISCLOSURE OF THE INVENTION

It has surprisingly been found that 5-HT₆ receptor antagonists, belonging to the class of sulfonamide compounds disclosed in WO01/32646 reduce food intake and body weight. Consequently, the present invention provides the use of a compound, which binds selectively to 5-HT₆-receptors, having a structure in accordance with formula (I) or a pharmaceutically acceptable salt thereof: 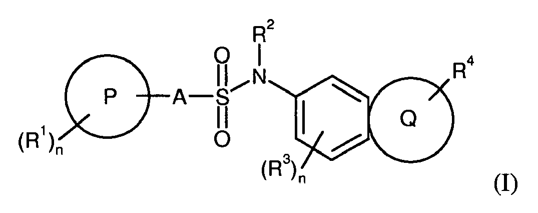 wherein
P is phenyl, naphthyl, a 5 or 6 membered heteroaryl ring comprising 1 to 3 heteroatoms selected from oxygen, nitrogen and sulfur, or a bicyclic or tricyclic heteroaryl ring comprising 1 to 3 heteroatoms selected from oxygen, nitrogen and sulfur;
A is a single bond, a C₁₋₆alkylene or a C₂₋₆alkenylene group;
R¹ is halogen, C₁₋₆alkyl optionally substituted by one or more halogen atoms, C₂₋₆cycloalkyl, phenyl, COC₁₋₆alkyl, C₁₋₆alkoxy, OCF₃, hydroxy, hydroxy-C₁₋₆alkyl, hydroxy-C₁₋₆alkoxy, C₁₋₆alkoxy- C₁₋₆alkoxy, nitro, amino, C₁₋₆alkylamino, or di-C₁₋₆alkylamino;
n is 0, 1, 2, 3, 4 or 5;
R² is hydrogen, C₁₋₆alkyl or together with a group R³ forms a group -(CR⁶R⁷)ₚ- where R⁶ and R⁷ are independently hydrogen or C₁₋₆alkyl and p is 2, 3 or 4;
R³ is C₁₋₆alkyl optionally substituted by one or more halogen atoms, halogen, C₁₋₆ alkoxy or together with the group R² forms a group -(CR⁶R⁷)ₚ- as defined above;
m is 0, 1 or 2;
R⁴ is a group -X-R⁵ where X is a single bond, CH₂, O, NH or N-C₁₋₆alkyl;
R⁵ is an optionally substituted 5- to 7-membered heterocyclic ring or a bicyclic heterocyclic ring comprising 1 to 3 heteroatoms selected from nitrogen, sulfur or oxygen; and
Q together with the phenyl group to which it is fused forms a quinoline, isoquinoline or quinazoline ring,
in the manufacture of a medicament for the treatment of obesity.

In some embodiments of the invention, the body weight disorder to be addressed is obesity in a human, defined as a condition where the individual has a Body Mass Index ("BMI"), sometimes called Quetelet's Index, above currently accepted standards. BMI is calculated by dividing weight (in kg) by height² (in meters²). The current standards for both men and women accepted as "normal" are a BMI of 20-24.9 kg/m². Grade I obesity corresponds to a BMI of 25-29.9 kg/m² ; Grade II obesity corresponds to a BMI of 30-40 kg/m² ; and Grade III obesity corresponds to a BMI greater than 40 kg/m². (E. Jequier, "Energy, obesity, and body weight standards," Am. J Clin. Nutr., 45:1035-47 (1987)). Ideal body weight will vary among species and individuals based on height, body build, bone structure, and sex.

The obesity herein may be due to any cause, whether genetic or environmental. Examples of causes that may result in obesity or be the cause of obesity include overeating, diet rich in fats or sugars, environmental causes, medications, pathological conditions showing reduced metabolic activity or a decrease in resting energy expenditure as a percentage of total fat-free mass. Such induction of body weight increase is particularly suited for treatment using the compounds and compositions resulting from the use or process of the invention, or alternatively the methods of the invention. In this context, "treatment" of a body weight disorder refers e. g., to a reduction of an abnormally or pathologically elevated body weight, or, to a reduction of an abnormally high rate of increase in body weight.

Thus, in one aspect, the invention relates to the use of a compound in accordance with formula (I) in the manufacture of a medicament for inhibition and/or complete suppression of lipogenesis in obese mammals, i.e., the excessive accumulation of lipids in fat cells, which is one of the major features of human and animal obesity, or loss of total body weight.

Another aspect of the invention is a use of a compound in accordance with formula (I) in the manufacture of a medicament for ameliorating the conditions that are a consequence of disease, such as preventing or arresting the progression of polycystic ovarian disease, so that the patient is no longer infertile, and increasing the insulin sensitivity and/or decreasing or eliminating the need or usage of insulin in a diabetic patient, e.g., one with adult-onset diabetes or Type II diabetes.

Still another aspect of the invention is a use of a compound in accordance with formula (I) in the manufacture of a medicament for reducing the food intake in mammals, including humans.

A further aspect of the invention is a cosmetic use of compounds of formula (I), as described herein, for causing loss of weight, as well as cosmetic compositions containing said compounds.

Yet another aspect of the invention is non-therapeutic method for improving the bodily appearance of a mammal, including a human, in which the method comprises orally administering to said mammal a compound of formula (I) as described herein.

"Treatment" (of obesity) refers to reducing the BMI of the mammal to less than about 25.9, and maintaining that weight for at least 6 months. The treatment suitably results in a reduction in food or calorie intake by the mammal.

"Prevention" (of obesity) refers to preventing obesity from occurring if the treatment is administered prior to the onset of the obese condition. Moreover, if treatment is commenced in already obese subjects, such treatment is expected to prevent, or to prevent the progression of, the medical sequelae of obesity, such as, e.g., arteriosclerosis, Type II diabetes, polycystic ovarian disease, cardiovascular diseases, osteoarthritis, dermatological disorders, hypertension, insulin resistance, hypercholesterolemia, hypertriglyceridemia, and cholelithiasis.

The term 'halogen' is used herein to describe, unless otherwise stated, a group selected from fluorine, chlorine, bromine or iodine.

The expression "C₁₋₆alkyl" includes methyl and ethyl groups, and straight-chained, branched or cyclic propyl, butyl, pentyl and hexyl groups. Particular alkyl groups are methyl, ethyl, n-propyl, isopropyl and tert-butyl. Derived expressions such as "C₁₋₆alkoxy" and "C₁₋₆ alkylamino" are to be construed accordingly.

The expression "C₁₋₆alkylene" as used herein refers to straight-chained and branched alkylene groups containing from 1 to 6 carbon atoms. Typical examples include methylene, ethylene, propylene and butylene groups.

The expression "C₂₋₆ alkenylene" as used herein refers to straight-chained and branched alkenylene groups containing from 2 to 6 carbon atoms. Typical examples include vinylene, allyl, dimethylallyl and butenylene groups.

The term "heteroaryl" refers to an aromatic 5-8 membered monocyclic, 8-12 membered bicyclic, or 11-14 membered tricyclic ring system having carbon atoms and 1-3 heteroatoms if monocyclic, 1-6 heteroatoms if bicyclic, or 1-9 heteroatoms if tricyclic, said heteroatoms selected from O, N, or S, wherein 0, 1, 2, 3, or 4 atoms of each ring may be substituted by a substituent.

The term "heterocyclic" refers to a nonaromatic 5-8 membered monocyclic, 8-12 membered bicyclic, or 11-14 membered tricyclic ring system having carbon atoms and 1-3 heteroatoms if monocyclic, 1-6 heteroatoms if bicyclic, or 1-9 heteroatoms if tricyclic, said heteroatoms selected from O, N, or S, wherein 0, 1, 2 or 3 atoms of each ring may be substituted by a substituent.

When P is naphthyl this is intended to denote both 1-naphthyl and 2-naphthyl groups. When P is a 5 or 6-membered heteroaryl ring suitable examples include thienyl, furyl, pyrrolyl, triazolyl, diazolyl, oxazolyl, thiazolyl, oxadiazolyl, isothiazolyl, isoxazolyl, thiadiazolyl, pyridyl, pyrimidyl and pyrazinyl. When P is a bicyclic heteroaryl ring suitable examples include indolyl, benzofuryl, benzothienyl, quinolinyl and isoquinolinyl. When P is a tricyclic heteroaryl ring a preferred example is dibenzofuryl. The heteroaryl rings can be linked to the remainder of the molecule via any suitable carbon atom or, when present, a nitrogen atom.

Preferably P is phenyl, naphthyl, benzofuryl or benzothienyl.

Preferably A is a single bond, a methylene or ethylene group or a -CH=CH group. Most preferably A is a single bond.

When n is more than 1 the groups R¹ can be the same or different. Preferably R¹ is halogen (particular chloro or bromo), or a C₁₋₆alkyl group optionally substituted by one or more halogen atoms, for example, methyl, ethyl, isopropyl, t-butyl or trifluoromethyl.

Preferably n is 0, 1, 2 or 3, particularly 1 or 2.

When R² together with a group R³ forms a further group -(CR⁶R⁷)ₚ- both of the groups R⁶ and R⁷ are preferably hydrogen and p is preferably 2.

R² is preferably hydrogen.

A substituent R³ can be attached at any unsubstituted carbon atom within the fused ring. When m is more than 1 the groups R³ can be the same or different. It will be appreciated that when the R²/R³ groups are linked together, the group R³ must be attached to one of the carbon atoms of the fused ring with an ortho relationship with respect to the sulfonamide linkage.

Preferably m is 0.

The group R⁴ can be attached at any unsubstituted carbon atom within the ring Q.

When R⁵ is a 5- to 7- membered heterocyclic ring suitable examples include piperazinyl, piperidyl, pyrrolidinyl and morpholinyl. The 5- to 7- membered heterocyclic rings can be linked to the remainder of the molecule via a carbon atom or, when present, a suitable nitrogen atom. It will be appreciated however, that when X is O, NH or N-C₁₋₆alkyl then the 5- to 7- membered heterocyclic ring must be linked to the rest of the molecule via a carbon atom. Preferably X is a single bond (i.e. R⁴ = R⁵) and the 5- to 7-membered heterocyclic ring is attached to the rest of the molecule via a suitable nitrogen atom.

When R⁵ is a bicyclic heterocyclic ring, X is preferably a single bond (i.e. R⁴ = R⁵) and suitable examples of such groups are

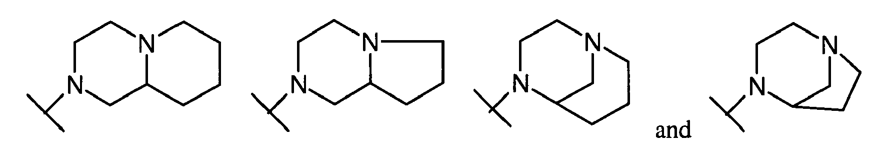

Optional substituents for rings within the definition of R⁵, which can be present on carbon and/or nitrogen atoms, include C₁₋₆alkyl, in particular methyl.

Most preferably R⁴ is an unsubstituted piperazine or N-methyl piperazine attached to the rest of the molecule via a suitable nitrogen atom.

Most preferably Q, together with the phenyl ring to which it is fused, forms a quinoline ring, and the substituent R⁴ is at the 4-position, that is to say, a group of formula (A)

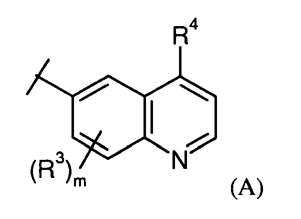

Particular preferred compounds of this invention include:
5-chloro-3-methyl-benzo[b]thiophene-2-sulfonic acid (4-[4-methylpiperazin-1-yl] quinolin-6-yl)amide,
5-chloro-naphthalene-2-sulfonic acid (4-[4-methyl-piperazin-1-yl]-quinolin-6-yl)amide,
4-bromo-*N*-[4-(4-methyl-piperazin-1-yl)-quinolin-6-yl]benzenesulfonamide,
3,5-dichloro-*N*-[4-(4-methyl-piperazin-1-yl)-quinolin-6-yl]benzenesulfonamide,
5-chloro-3-methyl-benzo[b]thiophene-2-sulfonic acid [4-(3,5-dimethylpiperazin-1-yl)-quinolin-6-yl] amide,
5-chloro-3-methyl-benzo[b]thiophene-2-sulfonic acid [4-(4-methyl-piperazin-1-yl)-quinazolin-6-yl]amide,
5-chloro-3-methyl-benzo[b]thiophene-2-sulfonic acid (4-piperazin-1-yl-quinolin-6-yl) amide,
3,5-dichloro-*N*-(4-piperazin-1-yl-quinolin-6-yl)benzenesulfonamide,
5-chloro-3-methyl-benzofuran-2-sulfonic acid (4-piperazin-1-yl-quinolin-6-yl)amide,
5,7-dichloro-3-methyl-benzo[b]thiophene-2-sulfonic acid (4-piperazin-1-yl-quinolin-6-yl)amide,
5-chloro-naphthalene-2-sulfonic acid (4-piperazin-1-yl-quinolin-6-yl)amide,
5-chloro-naphthalene-1-sulfonic acid (4-piperazin-1-yl-quinolin-6-yl)amide,
5-chloro-2-methyl- benzo[b]thiophene-3-sulfonic acid (4-piperazin-1-yl-quinolin-6-yl)amide,
2-dibenzofuran-sulfonic acid (4-piperazin-1-yl-quinolin-6-yl)amide,
5-chloro-3,7-dimethyl-benzo[b]thiophene-2-sulfonic acid (4-piperazin-1-yl-quinolin-6-yl)amide,
7-chloro-2-methyl-benzo[b]thiophene-3-sulfonic acid (4-piperazin-1-yl-quinolin-6-yl) amide,
4,6-dichloro-2-methyl-benzo[b]thiophene-3-sulfonic acid (4-piperazin-1-yl-quinolin-6-yl)amide,
5,7-dichloro-2-methyl-benzo[b]thiophene-3-sulfonic acid (4-piperazin-1-yl-quinolin-6-yl)amide,
biphenyl-4-sulfonic acid (4-piperazin-1-yl-quinolin-6-yl)amide,
4-*tert*-butyl-*N*-(4-piperazin-1-yl-quinolin-6-yl)benzenesulfonamide,
5-bromo-3-methyl-benzo[b]thiophene-2-sulfonic acid (4-piperazin-1-yl-quinolin-6-yl) amide,
4-*n*-butyl-*N*-(4-piperazin-1-yl-quinolin-6-yl)benzenesulfonamide,
4-chloro-2,5-dimethyl-*N*-(4-piperazin-1-yl-quinolin-6-yl)benzenesulfonamide,
5-chloro-3-ethyl-benzo[b]thiophene-2-sulfonic acid (4-piperazin-1-yl-quinolin-6-yl) amide,
5-chloro-3-isopropyl-benzo[b]thiophene-2-sulfonic acid (4-piperazin-1-yl-quinolin-6-yl)amide,
4-iodo-*N*-(4-piperazin-1-yl-quinolin-6-yl)benzenesulfonamide,
1-(5-chloro-3-methyl-benzo[b]thiophene-2-sulfonyl)-8-(4-methyl- piperazin-1-yl)-2,3-dihydro-1*H*-pyrrolo[2,3-g]quinoline,
5-chloro-naphthalene-2-sulfonic acid (2-methyl-4-piperazin-1-yl-quinolin-6-yl)amide,
5-chloro-3-methyl-benzo[b]thiophene-2-sulfonic acid (2-methyl-4-piperazin-1-yl-quinolin-6-yl)amide,
5-chloro-3-methyl-benzofuran-2-sulfonic acid (2-methyl-4-piperazin-1-yl-quinolin-6-yl)amide,
5-chloro-naphthalene-2-sulfonic acid (3-methyl-4-piperazin-1-yl-quinolin- 6-yl)amide,
5-chloro-3-methyl-benzo[b]thiophen-2-sulfonic acid (3-methyl-4-piperazin-1-yl-quinolin-6-yl)amide,
5,7-dichloro-3-methyl-benzo[b]thiophen-2-sulfonic acid (3-methyl-4- piperazin-1-yl-quinolin-6-yl)amide,
5-chloro-3-methyl-benzofuran-2-sulfonic acid (3-methyl-4-piperazin-1-yl-quinolin-6-yl)amide,
4-*tert*-butyl-*N*-[4-(s-hexahydro-pyrrolo[1,2-a]pyrazin-2-yl)-quinolin-6-yl-benzenesulfonamide,
5-chloro-3-methyl-benzo[b]thiophen-2-sulfonic acid [4-(S-hexahydro-pyrrolo[1,2a]pyrazin-2-yl)-quinolin-6-yl] amide,
5-chloro-2-methyl-benzo[b]thlophene-3-sulfonic acid (4-[3,5-dimethylpiperazin-1-yl]quinolin-6-yl)amide,
5-chloro-3-methyl-benzo[b]thiophene-2-sulfonic acid [4-((S)-3-methyl-piperazin-1-yl)-quinotin-6-yl]amide,
5-chloro-3-methyl-benzo[b]thiophene-2-sulfonic acid [4-((R)-3-methyl-piperazin-1-yl)-quinolin-6-yl] amide,
5-chloro-3-methyl-benzo[b]thiophene-2-sulfonic acid [4-((R)-3-isopropyl-piperazine- 1-yl)-quinolin-6-yl] amide,
5-chloro-3-methyl-benzo[b]thiophene-2-sulfonic acid [4-(*trans*-2,5-dimethyl-piperazine-1-yl)-quinolin-6-yl]amide,
5-chloro-3-methyl-benzo[b]thiophene-2-sulfonic acid [8-(4-methyl-piperazin-1-yl) naphthalen-2-yl]amide
or a pharmaceutically acceptable salt thereof.

For use in medicine, the salts of the compounds of formula I will be pharmaceutically acceptable salts. Other salts may, however, be useful in the preparation of the compounds of formula I or of their pharmaceutically acceptable salts. Suitable pharmaceutically acceptable salts of the compounds of formula I include acid addition salts which may, for example, be formed by mixing a solution of the compound according to the invention with a solution of a pharmaceutically acceptable acid such as for example maleic, hydrochloric, hydrobromic, phosphoric, acetic, fumaric, salicylic, citric, lactic, mandelic, tartaric and methanesulfonic.

Compounds of formula (I) may also form solvates such as hydrates, and the invention also extends to these forms. When referred to herein, it is understood that the term 'compound of formula (I)' also includes these forms.

Certain compounds of formula (I) are capable of existing in stereoisomeric forms including diastereomers and enantiomers and the invention extends to each of these stereoisomeric forms and to mixtures thereof including racemates. The different stereoisomeric forms may be separated one from the other by the usual methods, or any given isomer may be obtained by stereospecific or asymmetric synthesis. The invention also extends to any tautomeric forms and mixtures thereof.

The present invention includes within its scope the use of prodrugs of the compounds of formula I above. In general, such prodrugs will be functional derivatives of the compounds of formula I which are readily convertible in vivo into the required compounds of formula I. Conventional procedures for the selection and preparation of suitable prodrug derivatives are described, for example, in Design of Prodrugs, ed. H. Bundgaard, Elsevier, 1985.

The compounds of formula I, to be used according to the invention, can be prepared according to the methods described in WO01/32646.

### EXAMPLE: Effect of compounds on food intake in ob/ob mice

### Animals

Obese (ob/ob) mouse is selected as the primary animal model for screening as this mutant mouse consumes high amounts of food resulting in a high signal to noise ratio. To further substantiate and compare efficacy data, the effect of the compounds on food consumption is also studied in wild type (C57BL/6J) mice. The amount of food consumed during 15 hours of infusion of compounds is recorded.

Male mice (obese C57BL/6JBom-Lep^{ob} and lean wild-type C57B1/6JBom; Bomholtsgaard, Denmark) 8-9 weeks with an average body weight of 50 g (obese) and 25 g (lean) are used in all the studies. The animals are housed singly in cages at 23±1°C, 40-60 % humidity and have free access to water and standard laboratory chow. The 12/12-h light/dark cycle is set to lights off at 5 p.m. The animals are conditioned for at least one week before start of study.

### Compounds

The test compounds are dissolved in solvents suitable for each specific compound such as cyclodextrin, cyclodextrin/methane sulfonic acid, polyethylene glycol/methane sulfonic acid, or saline. Fresh solutions are made for each study. Doses of 30, 50 and 100 mg kg⁻¹day⁻¹ are used. The purity of the test compounds is of analytical grade.

### Minipump implantation

The animals are weighed at the start of the study and randomized based on body weight. Alzet osmotic minipumps (Model 2001D; infusion rate 8 µl/h) are used and loaded essentially as recommended by the Alzet technical information manual (Alza Scientific Products, 1997; Teeuwes and Yam, 1976). Continuous subcutaneous infusion with 24 hours duration is used. The minipumps are either filled with different concentrations of test compounds dissolved in vehicle or with only vehicle solution and maintained in vehicle pre-warmed to 37°C (approx. 1h). The minipumps are implanted subcutaneously in the neck/back region under short acting anesthesia (metofane/enflurane). This surgical procedure lasts approximately 5 min. It takes about 3 h to reach steady state delivery of the compound.

### Food intake measurements

The weights of the food pellets are measured at 5 p.m. and at 8 p.m. for two days before (baseline) and one day after the implantation of the osmotic minipumps. The weighing is performed with a computer assisted Mettler Toledo PR 5002 balance. Occasional spillage is corrected for. At the end of the study the animals are killed by neck dislocation and trunk blood sampled for later analysis of plasma drug concentrations.

### Determination of plasma concentration

The plasma sample proteins are precipitated with methanol, centrifuged and the supernatant is transferred to HPLC vials and injected into the liquid chromatography /mass spectrometric system. The mass spectrometer is set for electrospray positive ion mode and Multiple Reaction Monitoring.

A linear regression analysis of the standards forced through the origin is used to calculate the concentrations of the unknown samples.

### Statistical evaluation

Food consumption for 15 hours is measured for the three consecutive days and the percentage of basal level values is derived for each animal from the day before and after treatment. The values are expressed as mean±SD and mean±SEM from eight animals per dose group. Statistical evaluation is performed by Kruskal-Wallis one-way ANOVA using the per cent basal values. If statistical significance is reached at the level of p<0.05, Mann-Whitney U-test for statistical comparison between control and treatment groups is performed.

### Formulation

5-Chloro-3-methyl-benzo[b]thiophene-2-sulfonic acid (4-[4-methylpiperazin-1-yl] quinolin-6-yl)amide was weighted in and dissolved to half of its final volume with a stock solution of PEG400 and 1.0% Tween 80. 100 mM Sodium Acetate was added to a final concentration of 10 mM. Purified water was added almost to final volume. The pH of the solution was measured and adjusted with 1M HCl. Qs to calculated weight with purified water. The solution was filtered through a 0.45 µm syringe filter (Millex HV).

| *Composition:* | | | |
|---|---|---|---|
| Example 1 | 2.3 mg/ml | 6.9 mg/ml | 23.0 mg/ml |
| PEG 400 | 50% w/v | 50% w/v | 50% w/v |
| Tween 80 | 0.5 %w/v | 0.5%w/v | 0.5% w/v |
| Sodium Acetate | 10mM | 10mM | 10mM |

| *Properties:* | | | |
|---|---|---|---|
| pH | 5,3 | 5,2 | 5,2 |

### EXAMPLE 1

Effect on food intake of 5-chloro-3-methyl-benzo[b]thiophene-2-sulphonic acid (4-[4-methylpiperazin-1-yl]quinolin-6-yl)amide.

| | mg/kg/day | | **Css, tot** | | | % of basal level | | P | **Inhibition (%)** |
|---|---|---|---|---|---|---|---|---|---|
| Compound | Nominal | Corrected | **µM** | SEM | n | Mean | SEM | vs. Control | **Based on basal** |
| | | | | | | | | | |
| Vehicle | 0 | | | | 8 | 65,7 | 2,8 | | |
| EXAMPLE 1 | 10 | 10.4 | 1,00 | 0,04 | 8 | 62,5 | 6,4 | P<0.7 | **4,9** |
| EXAMPLE 1 | 30 | 32.2 | 1,62 | 0,13 | 6 | 49,3 | 8,0 | P<0.04 | **25,0** |
| EXAMPLE 1 | 100 | 99.1 | 1,81 | 0,15 | 7 | 49,9 | 6,7 | P<0.02 | **24,1** |
| mCCP | 10 | 10.9 | 0,61 | 0,04 | 8 | 31,2 | 4,7 | P<0.003 | **52,5** |

| | | | | | | | | | |
|---|---|---|---|---|---|---|---|---|---|
| mCPP: m-chlorophenylpiperazine (reference compound) Css, tot : total plasma exposure of the test compound and mCPP respectively at steady state | | | | | | | | | |

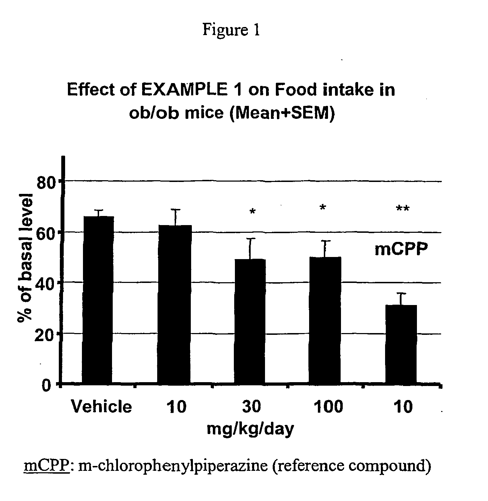

5-Chloro-3-methyl-benzo[b]thiophene-2-sulfonic acid (4-[4-methylpiperazin-1-yl] quinolin-6-yl)amide reduces food intake in ob/ob mice by 25% and 24 % at 30 and 100 mg/kg/day respectively, as shown in Figure 1.

### EXAMPLE 2

Effect on food intake of 4-*n*-butyl-N-(4-piperazin-1-yl-quinolin-6-yl)benzenesulphonamide.

| | mg/kg/day | | **Css, tot** | | | % of basal level | | P | **Inhibition (%)** |
|---|---|---|---|---|---|---|---|---|---|
| Compound | Nominal | Corrected | **µM** | SEM | n | Mean | SEM | vs. Control | **Based on basal** |
| | | | | | | | | | |
| Vehicle | 0 | | | | 8 | 65,2 | 4,9 | | |
| EXAMPLE 2 | 10 | 10,9 | 0,37 | 0,05 | 8 | 61,0 | 3,0 | P<0.40 | **6,5** |
| EXAMPLE 2 | 30 | 36,1 | 0,94 | 0,05 | 8 | 45,5 | 4,2 | P<0.005 | **30,2** |
| EXAMPLE 2 | 100 | 117,0 | 1,29 | 0,09 | 8 | 43,6 | 5,1 | P<0.002 | **33,2** |
| mCCP | 10 | 11,3 | 0,78 | 0,03 | 8 | 39,4 | 2,9 | P<0.002 | **39,6** |

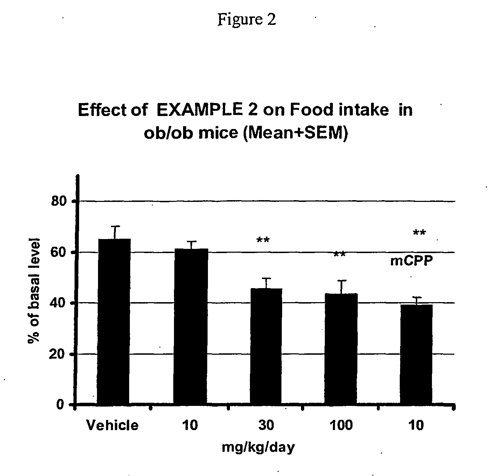

4-*n*-Butyl-*N*-(4-piperazin-1-yl-quinolin-6-yl)benzenesulfonamide reduces food intake in ob/ob mice by 30 % and 33 % at 30 and 100 mg/kg/day respectively, as shown in Figure 2.

## Claims

1. Use of a compound, which binds selectively to 5-HT₆-receptors, having a structure in accordance with formula (I) 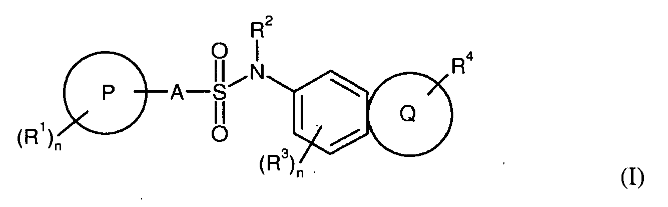 wherein
P is phenyl, naphthyl, a 5 or 6 membered heteroaryl ring comprising 1 to 3 heteroatoms selected from oxygen, nitrogen and sulfur, or a bicyclic or tricyclic heteroaryl ring comprising 1 to 3 heteroatoms selected from oxygen, nitrogen and sulfur;
A is a single bond, a C₁₋₆alkylene or a C₂₋₆alkenylene group;
R¹ is halogen, C₁₋₆alkyl optionally substituted by one or more halogen atoms, C₂₋₆cycloalkyl, phenyl, COC₁₋₆alkyl, C₁₋₆alkoxy, OCF₃, hydroxy, hydroxy-C₁₋₆alkyl, hydroxy-C₁₋₆alkoxy, C₁₋₆alkoxy- C₁₋₆alkoxy, nitro, amino, C₁₋₆alkylamino, or di-C₁₋₆alkylamino;
n is 0, 1, 2, 3, 4 or 5;
R² is hydrogen, C₁₋₆alkyl or together with a group R³ forms a group -(CR⁶R⁷)ₚ- where R⁶ and R⁷ are independently hydrogen or C₁₋₆alkyl and p is 2, 3 or 4;
R³ is C₁₋₆alkyl optionally substituted by one or more halogen atoms, halogen, C₁₋₆ alkoxy or together with the group R² forms a group -(CR⁶R⁷)ₚ- as defined above;
m is 0, 1 or 2;
R⁴ is a group -X-R⁵ where X is a single bond, CH₂, O, NH or N-C₁₋₆alkyl;
R⁵ is an optionally substituted 5- to 7-membered heterocyclic ring or a bicyclic heterocyclic ring comprising 1 to 3 heteroatoms selected from nitrogen, sulfur or oxygen; and
Q together with the phenyl group to which it is fused forms a quinoline, isoquinoline or quinazoline ring,
in the manufacture of a medicament for the treatment of obesity.

2. The use according to claim 1 in which said compound is in accordance with said formula (I) wherein P is phenyl, naphthyl, benzofuryl or benzothienyl.

3. The use according to claim 1 or 2 in which said compound is in accordance with said formula (I) wherein R¹ is halogen (particular chloro or bromo), or a C₁₋₆alkyl group optionally substituted by one or more halogen atoms.

4. The use according to any one of claims 1 to 3 in which said compound is in accordance with said formula (I) wherein R⁴ is a piperazine ring optionally substituted by C₁₋₆alkyl.

5. The use according to claim 1 in which the compound is selected from:
4-*tert*-butyl-*N*-(4-piperazin-1-yl-quinolin-6-yl)benzenesulfonamide,
5-chloro-3-methyl-benzo[b]thiophene-2-sulfonic acid (4-piperazin-1-yl-quinolin-6-yl) amide,

## Patentansprüche

1. Verwendung einer Verbindung, die selektiv an 5-HT₆-Rezeptoren bindet, mit einer Struktur gemäß der Formel (I) 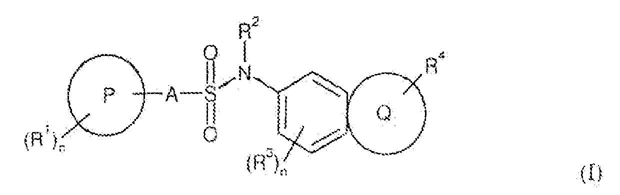 wobei
P Phenyl, Naphtyl, ein 5- oder 6-gliedriger Heteroarylring, umfassend 1 bis 3 aus Sauerstoff, Stickstoff und Schwefel ausgewählt Heteroatome, oder ein bicyclischer oder tricyclischer Hetereoarylring, umfassend 1 bis 3 aus Sauerstoff, Stickstoff und Schwefel ausgewählte Heteroatome, ist;
A eine Einfachbindung, eine C₁₋₆-Alkylen- oder eine C₂₋₆-Alkenylen-Gruppe ist;
R¹ Halogen, C₁₋₆-Alkyl, gegebenenfalls substituiert durch ein oder mehrere Halogenatome, C₂₋₆-Cycloalkyl, Phenyl, COC₁₋₆-Alkyl, C₁₋₆-Alkoxy, OCF₃, Hydroxy, Hydroxy-C₁₋₆-alkyl, Hydroxy-C₁₋₆-alkoxy, C₁₋₆-Alkoxy-C₁₋₆-alkoxy, Nitro, Amino, C₁₋₆-Alkylamino oder Di-C₁₋₆-alkylamino ist;
n 0, 1, 2, 3, 4 oder 5 ist;
R² Wasserstoff oder C₁₋₆-Alkyl ist oder zusammen mit einer Gruppe R³ eine Gruppe -(CR⁶R⁷)ₚ- bildet, worin R⁶ und R⁷ unabhängig voneinander Wasserstoff oder C₁₋₆-Alkyl sind und p 2, 3 oder 4 ist;
R³ C₁₋₆-Alkyl, gegebenenfalls substituiert durch ein oder mehrere Halogenatome, Halogen oder C₁₋₆-Alkoxy ist oder zusammen mit der Gruppe R² eine wie oben definierte Gruppe -(CR⁶R⁷)ₚ- bildet;
m 0, 1 oder 2 ist;
R⁴ eine Gruppe -X-R⁵ ist, worin X eine Einfachbindung, CH₂, O, NH oder N-C₁₋₆-Alkyl ist;
R⁵ ein gegebenenfalls substituierter 5- bis 7-gliedriger heterocyclischer Ring oder ein bicyclischer heterocyclischer Ring, umfassend 1 bis 3 aus Stickstoff, Schwefel oder Sauerstoff ausgewählte Heteroatome, ist; und
Q zusammen mit der Phenylgruppe, mit welcher diese fusioniert ist, einen Chinolin-, Isochinolin- oder Chinazolinring bildet,
zur Herstellung eines Arzneimittels für die Behandlung von Fettleibigkeit.

2. Verwendung nach Anspruch 1, in welcher die Verbindung der Formel (I) entspricht, wobei P Phenyl, Naphthyl, Benzofuryl oder Benzothienyl ist.

3. Verwendung nach Anspruch 1 oder 2, in welcher die Verbindung der Formel (I) entspricht, wobei R¹ Halogen (insbesondere Chlor oder Brom) oder eine gegebenenfalls durch ein oder mehrere Halogenatome substituierte C₁₋₆-Alkylgruppe ist.

4. Verwendung nach einem der Ansprüche 1 bis 3, in welcher die Verbindung der Formel (I) entspricht, wobei R⁴ ein gegebenenfalls durch C₁₋₆-Alkyl substituierter Piperazinring ist.

5. Verwendung nach Anspruch 1, in welcher die Verbindung ausgewählt ist aus:
4-*tert*-Butyl-*N*-(4-piperazin-1-yl-chinolin-6-yl)benzolsulfonamid, 5-Chlor-3-methylbenzo[b]thiophen-2-sulfonsäure(4-piperazin-1-yl-chinolin-6-yl)amid.

## Revendications

1. Utilisation d'un composé, qui se lie sélectivement aux récepteurs 5-HT₆, possédant une structure selon la formule (I) 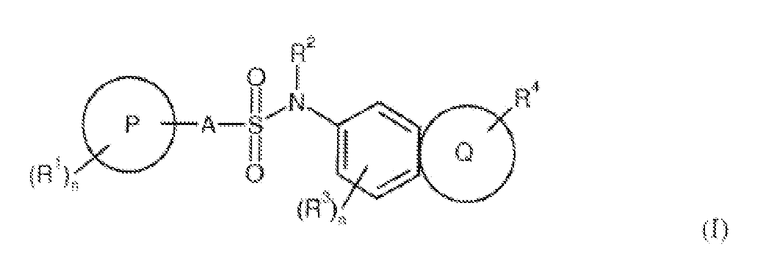 dans laquelle
P est un phényle, un naphtyle, un cycle hétéroaryle à 5 à 6 chaînons comprenant 1 à 3 hétéroatomes choisis parmi un atome d'oxygène, d'azote et de soufre, ou un cycle hétéroaryle bicyclique ou tricyclique comprenant 1 à 3 hétéroatomes choisis parmi un atome d'oxygène, d'azote et de soufre ;
A est une liaison simple, un groupe alkylène en C₁ à C₆ ou alcénylène en C₂ à C₆ ;
R¹ est un halogène, un alkyle en C₁ à C₆ facultativement substitué par un ou plusieurs atomes d'halogène, un cycloalkyle en C₂ à C₆, un phényle, CO-alkyle en C₁ à C₆, un alcoxy en C₁ à C₆, OCF₃, un hydroxy, un hydroxy-alkyle en C₁ à C₆, un hydroxy-alcoxy en C₁ à C₆, un (alcoxy en C₁ à C₆)-(alcoxy en C₁ à C₆), un nitro, un amino, un (alkyle en C₁ à C₆)-amino ou un di(alkyle en C₁ à C₆)-amino ;
n est 0, 1, 2, 3, 4 ou 5 ;
R² est un hydrogène, un alkyle en C₁ à C₆ ou ensemble avec un groupe R³ forme un groupe -(CR⁶R⁷)ₚ- où R⁶ et R⁷ sont indépendamment un hydrogène ou un alkyle en C₁ à C₆ et p est 2, 3 ou 4 ;
R³ est un alkyle en C₁ à C₆ facultativement substitué par un ou plusieurs atomes d'halogène, un halogène, un alcoxy en C₁ à C₆ ou ensemble avec le groupe R² forme un groupe -(CR⁶R⁷)ₚ- tel que défini ci-dessus ;
m est 0, 1 ou 2 ;
R⁴ est un groupe -X-R⁵ où X est une liaison simple, CH₂, O, NH ou N-alkyle en C₁ à C₆ ;
R⁵ est un cycle hétérocyclique à 5 à 7 chaînons facultativement substitué ou un cycle hétérocyclique bicyclique comprenant 1 à 3 hétéroatomes choisis parmi un atome d'azote, de soufre ou d'oxygène ; et
Q avec le groupe phényle auquel il est fusionné forme un cycle quinoline, isoquinoline ou quinazoline,
dans la fabrication d'un médicament destiné au traitement de l'obésité.

2. Utilisation selon la revendication 1, dans laquelle ledit composé est conforme à ladite formule (I) dans laquelle p est un phényle, un naphtyle, un benzofuryle ou un benzothiényle.

3. Utilisation selon la revendication 1 ou 2, dans laquelle ledit composé est conforme à ladite formule (I) dans laquelle R¹ est un halogène (en particulier un chloro ou un bromo), ou un groupe alkyle en C₁ à C₆ facultativement substitué par un ou plusieurs atomes d'halogène.

4. Utilisation selon l'une quelconque des revendications 1 à 3, dans laquelle ledit composé est conforme à ladite formule (I) dans laquelle R⁴ est un cycle pipérazine facultativement substitué par un alkyle en C₁ à C₆.

5. Utilisation selon la revendication 1, dans laquelle le composé est choisi parmi :
le 4-*tert*-butyl-*N*-(4-pipérazin-1-yl-quinolin-6-yl)benzènesulfonamide,
le (4-pipérazin-1-yl-quinolin-6-yl)amide de l'acide 5-chloro-3-méthyl-benzo [b]thiophène-2-sulfonique.
